# EUROPEAN PATENT SPECIFICATION

(11) **EP 1 337 237 B1**
(45) Date of publication and mention of the grant of the patent: **11.03.2009**
(21) Application number: 01996371.9
(22) Date of filing: 12.11.2001
(51) Int. Cl.: A61K 9/00, A61K 47/40

(54) **PHARMACEUTICAL PREPARATIONS COMPRISING CORTICOSTEROIDS AND ANTIINFECTIVE AGENTS**
PHARMAZEUTISCHE ZUSAMMENSETZUNGEN MIT CORTICOSTEROIDEN UND ANTIINFEKTIVA
PREPARATIONS PHARMACEUTIQUES CONTENANT DE CORTICOSTEROIDES ET D'AGENTS ANTIINFECTIVES

(30) Priority: 15.11.2000 IN MU101800; 09.11.2001 IN MU107701
(43) Date of publication of application: 27.08.2003
(73) Proprietor: Chandavarkar, Mohan A., Mumbai 400 050 (IN); Chandavarkar, Nandan M., Mumbai 400 050 (IN)
(72) Inventor: CHANDAVARKAR, Mohan, A., Mumbai 400 050 (IN); CHANDAVARKAR, Nandan, Mohan, Mumbai 400 050 (IN); CHANDRASHEKHAR, Mainde, Andheri (E), Mumbai 400 059 (IN)
(74) Representative: RACKETTE Partnerschaft Patentanwälte
(86) International application number: PCT/IN2001/000199
(87) International publication number: WO 2002/039993

(56) References cited:
- WO-A-90/01933
- US-A- 5 516 808
- US-A- 5 747 061
- H. A. DOSHI; ET AL.: "Preparation and evaluation of new eye-drops containing a combination of ciprofloxacin and dexamethasone" INDIAN DRUGS, vol. 37, no. 4, pages 190-195, XP002157878

## Description

**Field of Invention:** The invention relates to a combination drug comprising of anti-inflammatory agents in the nature of steroids and an anti- infective agent.

### Background of invention:

Anti-infective agents in the form of antibacterial agents and anti-inflammatory agents in the nature of steroids, have been used in combination for effective control of infection and inflammation. Use of such combination drugs of appropriate antibacterial agents and steroids helps in increasing patient compliance to therapy as it reduces the number of instillation of different drugs. This is specially true in the case of ophthalmic treatment, when the number of instillation of drugs requires to be minimal.

Not only is a combination of steroid and antibacterial agents desirable, but it is also essential that the combination when required for ophthalmic purposes is available as a clear formulation, either as a solution or a gel.

In formulations, for ophthalmic treatment, a clear formulation is highly desirable, because any formulation in suspension form causes irritation to the eye, as well as causes crust formation around the eye. The U.S. Patent No.5472954 describes a process by which the solubility of lipophilic or sparingly soluble active ingredients (drug/ cosmetic/ agrochemical) is increased by complexing with different derivatives of cyclodextrin. This patent only describes a process by which a single active ingredient can be made soluble by complexing it with cyclodextrin derivatives.

In post operative ophthalmic treatment, where in a combination therapy of antibiotic or antibacterial and anti- inflammatory agent is used, a clear formulation is highly desired. Even in non-operative cases ophthalmic preparations in clear formulation is preferred to a suspension, as a preferred line of treatment, as it increases the physical and physiological acceptance of the medication.

Ciprofloxacin, which is a quinolone derivative, and a broad spectrum antibacterial, and considered a 'standard' for the treatment of ocular bacterial infections is currently available in local markets in combination with the steroid dexamethasone in a suspension form. US Patent No: 5, 747, 061 describes an ophthalmic anti-inflamatory preparation comprising of corticosteroid with or without antibiotics in suspension form.

US Patent no: 5,516,808 again describes an antibiotic gel for non-opthalmic use comprising of one or more antibiotics with or without steroids like dexamethasone. This again is not a clear gel, and its preferred use is therefore for non-opthalmic purposes.

In addition U.S. Patent No: 6,284,804 describes a suspension formulation containing dexamethasone and ciprofloxacin together with a non-ionic polymer, non-ionic surfactant and an ionic tonicity agent.

Though the above combination is known to be effective against most ocular pathogens, it is not preferred for use in post- operative cases, because it is currently available only in suspension form. An instillation of a drug in suspension form results in crust formation around the eye causing a foreign bodylike feeling in the eye and irritation. Steroids are generally sparingly soluble in aqueous solution which is the reason for most anti-infective agent / steroid combinations being available in suspension form. The antibiotic and the steroid in the form of their salts are individually available as a clear solution, but the combination, does not give a stable clear solution. On standing turbidity appears in the mixture of antibiotic steroid solution.

Further a mere admixture of antibacterial agent in clear solution and the steroid in clear solution, usually results in the crystallization of the antibacterial because of the higher pH used to dissolve the steroid, and the steroid itself degrades in a short time when mixed with the antibacterial solution because of the lower pH used to dissolve the antibacterial agent.

H.A.Doshi et al (Indian Drugs 31 (4) April 2000), have described an invention for an ophthalmic preparation where in the principal ingredients are ciprofloxacin and dexamethasone along with Hydroxy Propyl Beta cylcodextrin as a solubiliser. An elevated temperature of 90 °C is used to dissolve dexamethasone in a buffer solution of Hydroxy Propyl Beta cyclodextrin.

Patent No: WO 90/01933 describes an invention for an ophthalmic preparation, consisting essentially of a broad spectrum quinalone and a steroid like dexamethasone. Although the invention refers to the use of co-solvents like cyclodextrin and viscosity agents like PVA, the invention does not mention a formulation where because of the critical ratio between the co-complexing agent and the PVA, a clear stable solution is obtained, which is a decided advantage in ophthalmic preparations.

The present invention describes for the first time a combination drug of a steroid and antibacterial, where in the activity and bio efficacy of the antibacterial and steroid are not in anyway hampered or affected. The invention also provides for the preparation of the combination drug in a clear solution or clear gel form, and is particularly ideal for ophthalmic treatment. The preparation causes a protective lubricating film over the cornea, giving the user a distinct advantage. The clear solution or clear gels of antibacterial and steroid, would be specially suitable as a preferred form of drug administration in post operative ophthalmic cases.

### SUMMARY:

In its main aspect the invention consists of a clear stable preparation of a combination drug, comprising of an anti inflammatory agent and an anti-infective agent. The anti-inflammatory agent in this invention is a corticosteroid , and the anti-infective agent is a derivative of quinolone , amino-glycoside or their pharmaceutically acceptable salts. The combination drug essentially comprises of i. An anti-inflammatory agent which is a corticosteroid, ii. An anti-infective agent selected from the group comprising of derivatives of quinolone, aminoglycoside and their pharmaceutically acceptable salts; iii.) β-cyclodextrin as a solubiliser, where in the solubiliser and steroid are in the ratio of 1M : 2M to IM : 6M to form a steroid solubiliser blend iv) a steroid solubiliser complexation enhancing polymer selected from among polyvinyl alcohol and polyvinyl pyrrolidone along with pharmaceutically acceptable excipients within a suitable carrier system.

In another aspect, this invention consists of a stable pharmaceutical preparation, which is a combination drug of a corticosteroid from the group consisting of fluorometholone, dexamethasone, Beta-methasone, corticosterone, prednisolone, and their derivatives essentially having a common nuclear structure Pregna-1,4-diene-3,20 dione. The anti-infective agents are selected from the group consisting of ciprofloxacin, norfloxacin, ofloxacin, sparfloxacin, tobramycin, gentamicin and their pharmaceutically acceptable derivatives and salts.

In a further aspect of this invention, the complexation enhancing polymer used is selected from the group consisting of non-ionic polymers like polyvinyl alcohol or polyvinyl pyrrolidone and its derivatives.

In yet another aspect of this invention, the combination drug is incorporated in a carrier system, which may be water, gel or ointment base.

In the final aspect of this invention, the combination drug when incorporated in a carrier system of water or gel, is a clear and stable pharmaceutical preparation, suitable for ocular treatment..

### DESCRIPTION

A brief description of the invention is as follows:

In its main embodiment, the invention consists of a stable pharmacuetical preparation, of a combination drug comprising of i..Anti-infective agents from the group consisting of quinolone derivatives, aminoglycoside derivatives and their pharmaceutically acceptable salts; ii Anti-inflammatory agents which are corticosteroids iii β-cyclodextrin as a solubiliser, where in the solubiliser and steroid are in the ration of 1M:2M to 1M:6M, to form a steroid solubiliser blend iv) a steroid -solubiliser complexation enhancing polymer; v) pharmaceutically acceptable excipients in a carrier system

The process of manufacturing this combination drug consists of the following steps:
i. Including the steroid within a solubiliser which exhibits an inclusion phenomena and together forming a solubiliser steroid blend. The solubiliser and steroid are taken in desired amounts usually ranging in the ratio between 1M : 2M to 1M : 6M and mixed thoroughly in a polybag to form a blend. The exact ratio in which the steroid and solubilizer are taken depends on the steroid and the anti-infective agent used. The solubiliser used is Beta cyclodextrin or its derivatives. The use of Beta cyclodextrin makes this process commercially cost effective.
ii. Dispersing or dissolving the anti-infective agent in a suitable solvent preferably water. The solvent and anti-infective agent are stirred to form a slurry. A complexation enhancing polymer solution capable of forming a protective lubricating film is added to the slurry and sufficient water is further added and stirred for 10 to 30 minutes to form a clear anti-infective agent polymer solution. Dispersion is usually carried out at a temperature between 20° to 50° C, preferably ambient temperature. The solution is maintained at a pH range between 4 to 7.
iii. Controlled addition of anti-infective agent polymer solution to steroid solubiliser blend with constant stirring to form a water soluble complex of anti-infective agent-steroid - polymer and solubiliser. The addition can even be done at room temperature. The clarity of the final solution is dependant on the crucial ratio of the anti-infective agent polymer solution to the steroid-solubiliser blend. The ratio depends upon the type of anti-infective agent and anti-inflammatory agent used. If the ratio is disturbed the clarity of the final solution is affected. The complexation usually occurs within 30 minutes. The complex formed is stable up to a temperature of 50°C.
iv. Incorporating the complex in a carrier system. The complex may be incorporated together with suitable excipients, in water , ointment base or a gel, depending upon the final purpose of its use.

The complex when dissolved in water or gel , along with excipients, forms a clear stable solution and clear stable gel respectively, which is very suitable for ocular treatment. The excipients used are benzalkonium chloride as preservative, disodium EDTA as chelating agent, mono or dibasic sodium phosphate as a buffering agent, and sodium chloride as tonicity adjustor. In respect of gel preparations sorbitol is used as tonicity adjustor.

In its preferred embodiment the steroids used are selected from the group having a general nuclear structure Pregna - 1, 4 - diene - 3, 20 dione which could be dexamethasone, fluorometholone, Betamethasone, corticosterone, prednisolone and such others. The anti-infective agents are selected from the group consisting of derivatives of Ciprofloxacin, Ofloxacin, Sparfloxacin, Norfloxacin, Tobramycin sulphate, Gentamicin sulphate and their pharmceutically acceptable salts.

The following examples specifically describe the various combination drugs and the process for the different combinations of steroids and anti-infective agents used in the various carrier systems.

### MANUFACTURING PROCESS OF THE EYE DROPS:

### Example : 1

### Steroid - Dexamethasone

### Anti-infective agent - Ciprofloxacin Hydrochloride

1. 0.1% of Dexamethasone and 1% of Betacyclodextrin are accurately weighed.
2. Betacyclodextrin and Dexamethasone are blended thoroughly for 5 minutes.
3. 0.35% of Ciprofloxacin Hydrochloride is dispersed in water and stirred for 5 minutes to form a slurry
4. 1% of Polyvinyl alcohol solution is slowly added as a complexation enhancing agent. Sufficient water is added and stirred for 10-30 minutes to form a clear solution.
5. Blend of solubilized Dexamethasone and Betacyclodextrin are gradually added to Step No. 4 by stirring it for 10-30 minutes.
6. 0.0005% of Sodium Phosphate is added to Step 5 as a buffering agent and stirred for 5 minutes.
7. 0.1% of Disodium Edetate is added to Step No. 6 as a chelating agent.
8. 0.01% of Benzalkonium Chloride is added as a preservative to Step No. 7.
9. 0.8% of Sodium Chloride is added to Step No. 8 as a tonicity adjustor.
10. The solution is stirred for 10 minutes and pH of the solution is checked. Sodium Hydroxide solution is used to adjust the pH of solution between 4.5 and 5.0.
   * All the steps from 1 to 9 are carried out in class 100 area.
11. The solution is filtered through 0.2 µ filter to get a sterile ophthalmic solution. The solution is filled in a suitable container.

### Example: 2

### Steroid - Dexamethasone

### Anti-infective agent - Tobramycin Sulphate

1. 0.1% of Dexamethasone and 1% of Betacyclodextrin are accurately weighed.
2. Betacyclodextrin and Dexamethasone are blended thoroughly for 5 minutes.
3. 0.3% of Tobramycin Sulphate is dispersed in water and stirred for 5 minutes to form a slurry
4. 1% of Polyvinyl alcohol solution is slowly added as a co-complexing agent. Sufficient water is added and stirred for 10-30 minutes to form a clear solution.
5. Blended solublized steriod of Dexamethasone and Betacyclodextrin are gradually added to Step No. 4 by stirring it for 10-30 minutes.
6. 0.0005% of Sodium Phosphate is added to Step 5 as a buffering agent.
7. 0.1% of Disodium Edetate is added to Step No. 6 as a chelating agent.
8. 0.01% of Benzalkonium Chloride is added as a preservative to Step No. 7.
9. 0.8% of Sodium Chloride is added to Step No. 8 as a tonicity adjustor.
10. The solution is stirred for 10 minutes and pH of the solution is checked. Sodium Hydroxide solution is used to adjust the pH of solution between 7.0 and 7.5.
   * All the steps from 1 to 9 are carried out in class 100 area.
11. The solution is filtered through 0.2 µ filter to get a sterile ophthalmic solution. The solution is filled in a suitable container.

### Example: 3

### Steroid - Dexamethasone

### Anti-infective agent - Ofloxacin

1. 0.1 % of Dexamethasone and 1% of Betacyclodextrin are accurately weighed.
2. Betacyclodextrin and Dexamethasone are blended thoroughly for 5 minutes.
3. 0.3% of Ofloxacin is dispersed in water and stirred for 5 minutes to form a slurry
4. 1% of Polyvinyl alcohol solution is slowly added as a co-complexing agent. Sufficient water is added and stirred for 10-30 minutes to form a clear solution.
5. Blend of solublized Dexamethasone and Betacyclodextrin is gradually added to Step No. 4 by stirring it for 10-30 minutes.
6. 0.0005% of Sodium Phosphate is added to Step 5 as a buffering agent and stirred for 5 minutes.
7. 0.1 % of Disodium Edetate is added to Step No. 6 as a chelating agent.
8. 0.01% of Benzalkonium Chloride is added as a preservative to Step No. 7.
9. 0.8% of Sodium Chloride is added to Step No. 8 as a tonicity adjustor.
10. The solution is stirred for 10 minutes and pH of the solution is checked. Sodium Hydroxide solution is used to adjust the pH of solution between 6.5 and 7.
   * All the steps from 1 to 9 are carried out in class 100 area.
11. The solution is filtered through 0.2 µ filter to get a sterile ophthalmic solution. The solution is filled in a suitable container.

### Example : 4

### Steroid - Dexamethasone

### Anti-infective agent - Sparfloxacin

1. 0.1 % of Dexamethasone and 1 % of Betacyclodextrin are accurately weighed.
2. Betacyclodextrin and Dexamethasone are blended thoroughly for 5 minutes.
3. 0.3% of Sparfloxacin is dispersed in water and stirred for 5 minutes to form a slurry
4. 1% of Polyvinyl alcohol solution is slowly added as a complexing agent. Sufficient water is added and stirred for 10-30 minutes to form a clear solution.
5. Blended solubilized steriod of Dexamethasone and Betacyclodextrin are gradually added to Step No. 4 by stirring it for 10-30 minutes.
6. 0.0005% of Sodium Phosphate is added to Step 5 as a buffering agent and stirred for 5 minutes.
7. 0.1% of Disodium Edetate is added to Step No. 6 as a chelating agent.
8. 0.01% of Benzalkonium Chloride is added as a preservative to Step No. 7.
9. 0.8% of Sodium Chloride is added to Step No. 8 as a tonicity adjustor.
10. The solution is stirred for 10 minutes and pH of the solution is checked. Sodium Hydroxide solution is used to adjust the pH ;
   * All the steps from 1 to 9 are carried out in class 100 area.
11. The solution is filtered through 0.2 µ filter to get a sterile ophthalmic solution. The solution is filled in a suitable container.

### Example: 5

### Steroid-Dexamethasone

### Anti-infective agent - Gentamicin Sulphate

1. 0.1% of Dexamethasone and 1% of Betacyclodextrin are accurately weighed.
2. Betacyclodextrin and Dexamethasone are blended thoroughly for 5 minutes.
3. 0.3% of Gentamicin Sulphate is dispersed in water and stirred for 5 minutes to form a slurry
4. 1% of Polyvinyl alcohol solution is slowly added as a co-complexing agent. Sufficient water is added and stirred for 10-30 minutes to form a clear solution.
5. Blend of solublized Dexamethasone and Betacyclodextrin is gradually added to Step No. 4 by stirring it for 10-30 minutes.
6. 0.0005% of Sodium Phosphate is added to Step 5 as a buffering agent and stirred for 5 minutes.
7. 0. 1% of Disodium Edetate is added to Step No. 6 as a chelating agent.
8. 0.01% of Benzalkonium Chloride is added as a preservative to Step No. 7.
9. 0.8% of Sodium Chloride is added to Step No. 8 as a tonicity adjustor.
10. The solution is stirred for 10 minutes and pH of the solution is checked. Sodium Hydroxide solution is used to adjust the pH of solution between 4.5 and 5.0.
   * All the steps from 1 to 9 are carried out in class 100 area.
11. The solution is filtered through 0.2 µ filter to get a sterile ophthalmic solution. The solution is filled in a suitable container.

### Example 6:

### Steroid: Dexamethasone

### Anti-infective Agent: Norfloxacin

1. Accurately weigh 0.1% of Dexamethasone and 1% of Betacyclodextrin .
2. Blend thoroughly Betacyclodextrin and Dexamethasone for 5 minutes.
3. Disperse 0.3% of Norfloxacin in water to form a slurry and stir for 5 minutes.
4. Add 1% of Polyvinyl alcohol solution as a complexing agent slowly. Add sufficient water and stir for 10 - 30 minutes to form a clear solution.
5. Add solubilized steroid blend of Dexamethasone and Betacyclodextrin to Step No. 4 gradually under stirring for 10 - 30 minutes.
6. Add 0.0005% Sodium Phosphate as a buffering agent to Step No. 5 and stir for 5 minutes.
7. Add 0.1% of Disodium Edetate in Step No. 6 as chelating agent.
8. Add 0.01% of Benzalkonium Chloride as preservative in Step No. 7.
9. Add 0.8% of Sodium Chloride in Step No. 8 as a tonicity adjustor.
10. Stir the solution for 10 minutes and check pH of the solution. Adjust the pH of solution between 4.5 to 5.0 using Sodium Hydroxide solution.
   * Carry out all the steps 1 to 9 in class 100 area.
11. Filter the solution through 0.2 µ filter to get a sterile ophthalmic solution. Fill the solution in suitable container.

### Example 7:

### Steroid: Dexamethasone

### Anti-infective Agent: Ciprofloxacin

### Polymer: Polyvinyl Pyrollidone (PVP)

1. Accurately weigh 1% of Dexamethasone and 1% of Betacyclodextrin.
2. Blend thoroughly Betacyclodextrin and Dexamethasone for 5 minutes.
3. Disperse 0.35% of Ciprofloxacin Hydrochloride in water to form a slurry and stir for 5 minutes.
4. Add 1% of Polyvinyl Pyrollidone (PVP) solutions as a co-complexing agent slowly. Add sufficient water and stir for 10-30 minutes to form a clear solution.
5. Add solubilized blend of Dexamethasone and Betacyclodextrin to Step No.4 gradually under stirring for 10 -30 minutes.
6. Add 0.0005% Sodium Phosphate as a buffering agent to Step No.5 and stir for 5 minutes.
7. Add 0.1% of Disodium Edetate in Step No.6 as chelating agent.
8. Add 0.01% of Benzalkonium Chloride as preservative in Step No.7
9. Add 0.8% of Sodium Chloride in Step No.8 as a tonicity adjustor.
10. Stir the solution for 10 minutes and check pH of the solution. Adjust the pH of solution between 4.5 to 5.0 using Sodium Hydroxide solution.
   • Carry out all the steps 1 to 9 in class 100 area.
11. Filter the solution through 0.2 u filter to get a sterile ophthalmic solution. Fill the solution in suitable container.

### MANUFACTURING PROCESS OF OPHTHALMIC GEL:

### Example : 8

### Steroid - Dexamethasone

### Anti-infective agent - Ciprofloxacin Hydrochloride

1. 0.1% of Dexamethasone and 1% of Betacyclodextrin are accurately weighed.
2. Betacyclodextrin and Dexamethasone are blended thoroughly for 5 minutes.
3. 0.3% of Ciprofloxacin Hydrochloride is dispersed in water to form a solution and stirred for 5 minutes.
4. Blend of solublized Dexamethasone and Betacyclodextrin is gradually added by stirring it for 10-30 minutes.
5. 0.01 % of Benzalkonium Chloride is dissolved as a preservative in hot water and added to Step No. 4.
6. The solution of step 5 is filtered through 0.2 µ membrane filter and given a wash with water.
7. HPMC 2% is dispersed as viscofying agent in hot water with continuous stirring till a uniform gel without any lumps formation.
8. 12% of sorbitol is added to Step 6 as tonicity adjustor / Humactant under constant stirring.
9. The gel is autoclaved at 121° C for about 15 minutes.
10. The autoclaved gel is aseptically mixed (at room temperature) with the solution from Step 6 under continuous stirring till clear gel forms.

### MANUFACTURING PROCESS OF OPHTHALMIC GEL:

### Example : 9

### Steroid - Dexamethasone

### Anti-infective agent - Tobramycin Sulphate

1. 0.1% of Dexamethasone and 1% of Betacyclodextrin are accurately weighed.
2. Betacyclodextrin and Dexamethasone are blended thoroughly for 5 minutes.
3. 0.3% of Tobramycin Sulphate is dispersed in water to form a solution and stirred for 5 minutes.
4. Blend of solublized Dexamethasone and Betacyclodextrin is gradually added by stirring it for 10-30 minutes.
5. 0.01% of Benzalkonium Chloride is dissolved as a preservative in hot water and added to Step No. 4.
6. The solution of step 5 is filtered through 0.2 µ membrane filter and given a wash with water.
7. HPMC 2% is dispersed as viscofying agent in hot water with continuous stirring till a uniform gel without any lumps formation.
8. 12% of sorbitol is added to Step 6 as tonicity adjustor / Humactant under constant stirring.
9. The gel is autoclaved at 121° C for about 15 minutes.
10. The autoclaved gel is aseptically mixed (at room temperature) with the solution from Step 6 under continuous stirring till clear gel forms.

## Claims

1. A *clear* stable pharmaceutical preparation of a combination drug, comprising amongst others of:
(i) An anti-infective agent, selected from the group consisting of quinolone derivatives, amino-glycoside derivatives and their pharmaceutically acceptable salts;
(ii) An ant-inflammatory agent which is a corticosteroid;
(iii) β-cylcodextrin as a solubiliser, where in the solubiliser and steroid are in the ratio of 1M:2M to IM :6M, to form a steroid solubiliser blend;
*(iv)* A steroid solubilser complexation enhancing polymer, selected from among polyvinyl alcohol and polyvinyl pyrrolidone.
(v) pharmaceutically acceptable excipients within a suitable carrier system.

2. A preparation as claimed in claim 1, where in the steroid has a common nuclear structure Pregna-1, 4-diene-3, 20 dione.

3. A preparation as claimed in claim 2, where in the anti-inflammatory agent is a corticosteroid selected from the group consisting of fluorometholone, Beta-Methasone, prednisolone, dexamethasone and their derivatives;

4. A preparation as claimed in claim 3 where in the anti-infective agent is a quinolone derivative selected from the group consisting of ciprofloxacin, norfloxacin, ofloxacin, sparfloxacin and their pharmaceutically acceptable salts.

5. A preparation as claimed in claim 4, where in the anti-infective agent is an aminoglycoside derivative selected from amongst tobramycin, gentamicin and its pharmaceutically acceptable salts, preferably sulphates.

6. A preparation as claimed in claim 5, where in the preparation is incorporated in a carrier system consisting of water and excipients resulting in a clear stable solution for ocular treatment.

7. A preparation as claimed in claim 6, where in the preparation is incorporated in a carrier having gelling polymer, and excipients resulting in a clear stable gel.

8. A preparation as claimed in claim 6 where in the preparation is incorporated in a carrier consisting of hydrophobic ointment base and excipients

9. A preparation as claimed in claim 6,7 and 8 where the excepients used are a buffering agent, chelating agent, preservative and a tonicity adjustor.

10. A preparation as claimed in claim 9, where in the excipients are benzalkonium choride as preservatives, Edetate disodium (EDTA) as chelating agent, mono or dibasic sodium phosphate as a buffering agent and sodium chloride as tonicity adjustor, all in pharmaceutically acceptable concentrations.

11. A preparation as claimed in claim 10 , where in the carrier system is water and the sodium chloride is in amounts sufficient to cause the composition to have an osmolality of about 270 -320 MOSM.

12. A preparation as claimed in claim 11, where in the concentrations of the anti-infective agent ranges from 0.3% to 0.5% wt by volume and steriod ranges from 0.1% to 0.3% wt by volume in the final preparation.

13. A method to manufacture a preparation as described in claim 12, by a process essentially consisting of:
(a)Including the steroid within a complexation forming solubilizer , to form a solubilser-steroid blend;
(b) Dispersing or dissolving the anti-infective agent in a suitable solvent, which is subsequently diluted with a complexation enhancing water soluble polymer solution capable of causing a lubricating film to give an anti-infective agent - polymer solution;
(c)Combining the anti-infective agent polymer solution and steroid solubiliser blend to form a water soluble complex ;
(d) Incorporating the complex in a pharmaceutically acceptable carrier system; to give a stable pharmaceutical preparation.

14. A process as claimed in claim 13, wherein the solvent used for dissolving or dispersing anti-infective agent is water.

15. A process as claimed in claim 14, wherein the complexation enhancing polymer used is non-ionic, selected from the group consisting of polyvinyl alcohol or polyvinyl pyrrolidone, and its derivatives.

16. A process as claimed in claim 15, where in the water soluble complex is lyophilised.

17. A process as claimed in claim 16, where in the lyophilised complex is incorporated in a suitable carrier system.

18. A process as claimed in claim 15 or 17 , where in the carrier system is water with suitable excipients resulting in a clear pharmaceutical preparation for ocular treatment.

19. A process as claimed in claim 15 or 17 , where in the carrier consists of water, gelling polymer and excipients.

20. A process as claimed in claim 19 , where in the gelling polymer is selected from among hydroxy propyl, methyl cellulose, carbomer or its derivatives, carboxy methyl cellulose or methyl cellulose or hydroxyethyl cellulose.

21. A process as claimed in claim 15 or 17 , where in the carrier consists of ointment base and excipients.

## Patentansprüche

1. Klare stabile pharmazeutische Darstellung eines Kombinationsarzneimittels mit unter anderen folgenden Bestandteilen:
(i) einem infektionshemmenden Wirkstoff, der aus der Gruppe ausgewählt ist, die aus Quinolon-Derivaten, Aminoglykosid-Derivaten und deren pharmazeutisch verträglichen Salzen besteht,
(ii) einem entzündungshemmenden Wirkstoff, der ein Corticosteroid ist,
(iii) β-Zyklodextrin als Lösungsvermittler, wobei das Verhältnis zwischen Lösungsvermittler und Steroid zwischen 1M:2M und 1 M:6M liegt, wobei eine Mischung von Lösungsvermittler und Steroid ausgebildet ist,
(iv) einem die Komplexierung von Steroid und Lösungsvermittler fördernden Polymer, das unter Polyvinylalkohol und Polyvinylpyrrolidon ausgewählt ist,
(v) pharmazeutisch verträglichen Hilfsstoffen in einem geeigneten Trägersystem.

2. Darstellung nach Anspruch 1, wobei das Steroid eine gemeinsame Kernstruktur Pregna-1,4-dien-3,20-dion aufweist.

3. Darstellung nach Anspruch 2, wobei der entzündungshemmende Wirkstoff ein Corticosteroid aufweist, das aus der Gruppe ausgewählt ist, die aus Fluorometholon, Beta-Methason, Prednisolon, Dexamethason und deren Derivaten besteht.

4. Darstellung nach Anspruch 3, wobei der infektionshemmende Wirkstoff ein Quinolon-Derivat aufweist, das aus der Gruppe ausgewählt ist, die aus Ciprofloxacin, Norfloxacin, Ofloxacin, Sparfloxacin und deren pharmazeutisch verträglichen Salzen besteht.

5. Darstellung nach Anspruch 4, wobei der infektionshemmende Wirkstoff ein Aminoglykosid-Derivat aufweist, das unter Tobramycin, Gentamicin und deren pharmazeutisch verträglichen Salzen vorzugsweise Sulfaten ausgewählt ist.

6. Darstellung nach Anspruch 5, wobei die Darstellung in ein Trägersystem eingebettet ist, das aus Wasser und Hilfsstoffen besteht, so dass eine klare und stabile Lösung für die okulare Behandlung bereitgestellt ist.

7. Darstellung nach Anspruch 6, wobei in der Darstellung ein Träger mit einem gelierenden Polymer und Hilfsstoffe eingebettet sind, so dass ein klares stabiles Gel bereitgestellt ist.

8. Darstellung nach Anspruch 6, wobei in der Darstellung ein Träger eingebettet ist, der aus einer hydrophoben Salbenbasis und Hilfsstoffen besteht.

9. Darstellung nach Anspruch 6, 7 und 8, wobei die verwendeten Hilfsstoffe ein Puffermittel, ein Chelatbildner, Konservierungsmittel und ein Tonizitätssteller sind.

10. Darstellung nach Anspruch 9, wobei die Hilfsmittel Benzalkoniumchlorid als Konservierungsmittel, Edetatedinatrium (EDTA) als Chelatbildner, ein- oder zweibasisches Natriumphosphat als Puffermittel und Natriumchlorid als Tonizitätssteller sind, die alle beliebige pharmazeutisch verträgliche Konzentrationen aufweisen.

11. Darstellung nach Anspruch 10, wobei das Trägersystem Wasser ist und Natriumchlorid in ausreichender Menge vorliegt, um eine Osmolarität von etwa 270 bis 320 MOSM bereitzustellen.

12. Darstellung nach Anspruch 11, wobei die Konzentrationen des infektionshemmenden Wirkstoffes in der End-Darstellung von 0,3 Volumenprozent bis 0,5 Volumenprozent und diejenigen des Steroids von 0,1 Volumenprozent bis 0,3 Volumenprozent reichen.

13. Methode zum Herstellen einer Darstellung nach Anspruch 12 mittels eines Verfahrens mit den Schritten:
(a) Einbetten des Steroids in einen komplexierenden Lösungsvermittler unter Ausbildung einer Lösungsvermittler-Steroid-Mischung,
(b) Dispergieren oder Lösen des infektionshemmenden Wirkstoffes in einem geeigneten Lösungsmittel, das anschließend mit einer die Komplexierung fördernden wasserlöslichen Polymerlösung verdünnt wird, die zum Erzeugen eines Gleitfilms eingerichtet ist, so dass eine Lösung aus infektionshemmendem Wirkstoff und Polymer bereitgestellt ist,
(c) Kombinieren der Lösung aus infektionshemmendem Wirkstoff und Polymer mit der Lösungsvermittler-Steroid-Mischung unter Ausbildung eines wasserlöslichen Komplexes,
(d) Einbetten des Komplexes in ein pharmazeutisch verträgliches Trägersystem zum Bereitstellen einer stabilen pharmazeutischen Darstellung.

14. Verfahren nach Anspruch 13, wobei das Lösungsmittel zum Auflösen oder Dispergieren des infektionshemmenden Wirkstoffs Wasser ist.

15. Verfahren nach Anspruch 14, wobei das verwendete die Komplexierung fördernde Polymer nicht ionisch und aus der Gruppe ausgewählt ist, die aus Polyvinylalkohol, Polyvinylpyrrolidon und deren Derivaten besteht.

16. Verfahren nach Anspruch 15, wobei der wasserlösliche Komplex gefriergetrocknet wird.

17. Verfahren nach Anspruch 16, wobei der gefriergetrocknete Komplex in ein geeignetes Trägermaterial eingebettet wird.

18. Verfahren nach Anspruch 15 oder 17, wobei das Trägersystem Wasser mit geeigneten Hilfsstoffen ist, so dass eine klare pharmazeutische Darstellung für die okulare Behandlung bereitgestellt ist.

19. Verfahren nach Anspruch 15 oder 17, wobei der Träger Wasser, ein gelierendes Polymer und Hilfsstoffe aufweist.

20. Verfahren nach Anspruch 19, wobei das gelierende Polymer unter Hydroxypropyl, Methylzellulose, Carbomer oder deren Derivaten, Carboxymethylzellulose, Methylzellulose oder Hydroxyethylzellulose ausgewählt ist.

21. Verfahren nach Anspruch 15 oder 17, wobei der Träger aus einer Salbenbasis und Hilfsstoffen besteht.

## Revendications

1. Préparation pharmaceutique stable et claire d'une combinaison de médicaments comprenant entre autres :
(i) un agent anti-infectieux choisi dans le groupe consistant en des dérivés quinolones, des dérivés amino-glycosides et leurs sels pharmaceutiquement acceptables,
(ii) un agent anti-inflammatoire qui est un corticostéroïde,
(iii) de la β-cylcodextrine comme solubilisant, dans laquelle le solubilisant et le stéroïde sont de le rapport de 1 M:2M à 1 M:6M, pour former un mélange solubilisant stéroïde,
(iv) un polymère augmentant la complexation du solubilisant stéroïde, choisi dans le groupe les alcools poly-vinyliques et les pyrrolidones poly-vinyliques
(v) des excipients pharmaceutiquement acceptables à l'intérieur d'un système support convenable.

2. Préparation selon la revendication 1, dans laquelle le stéroïde a une structure nucléaire commune pregna-1, 4-diène-3, 20 dione.

3. Préparation selon la revendication 2, dans laquelle l'agent anti-inflammatoire est un corticostéroïde choisi dans le groupe comprenant la fluorométholone, la béta-méthasone, la prédnisolone, la dexaméthasone et leurs dérivés.

4. Préparation selon la revendication 3, dans laquelle l'agent anti-infectieux est un dérivé de quinolones choisi dans le groupe consistant en la ciprofloxacine, la norfloxacine, l'ofloxacine, la sparfloxacine et leurs sels pharmaceutiquement acceptables.

5. Préparation selon la revendication 4, dans laquelle l'agent anti-infectieux est un dérivé amino-glycoside choisi parmi la tobramycine, la gentamicine et ses sels pharmaceutiquement acceptables, de préférence les sulfates.

6. Préparation selon la revendication 5, dans laquelle la préparation est incorporée dans un système support comprenant de l'eau et des excipients donnant une solution stable claire pour traitement oculaire.

7. Préparation selon la revendication 6, dans laquelle la préparation est incorporée dans un support comprenant un polymère gélifiant et des excipients pour donner un gel stable clair.

8. Préparation selon la revendication 6, dans laquelle la préparation est incorporée dans un support consistant en un onguent hydrophobe et des excipients.

9. Préparation selon l'une des revendications 6, 7 et 8, dans laquelle les excipients utilisés sont un agent tampon, un agent chélatant, un conservateur et un ajusteur de tonicité.

10. Préparation selon la revendication 9, dans laquelle les excipients sont du chlorure de benzalkonium comme conservateur, de l'édétate de disodium (EDTA) comme agent chélatant, du phosphate mono ou dibasique de sodium comme agent tampon et du chlorure de sodium comme ajusteur de tonicité, tous dans des concentrations pharmaceutiquement acceptables.

11. Préparation selon la revendication 10, dans laquelle le système porteur est de l'eau et le chlorure de sodium est en quantités suffisantes pour conférer à la composition une osmolalité d'environ 270 - 320 MOSM.

12. Préparation selon la revendication 11, dans laquelle la concentration de l'agent anti-infectieux s'étend entre 0.3 % à 0.5 % en poids par volume et les stéroïdes s'étendent de 0.1 % à 0.3 % en volume dans la préparation finale.

13. Procédé de fabrication de la préparation telle que décrite dans la revendication 12 par un procédé consistant essentiellement à :
(a) incorporer le stéroïde dans un solubilisant formant un complexant, pour former un mélange stéroïde-solubilisant ;
(b) disperser ou dissoudre l'agent anti-infectieux dans un solvant convenable, qui est ensuite dilué avec une solution de polymère soluble dans l'eau et augmentant la complexation, apte à créer un film lubrifiant, pour donner une solution agent anti-infectieux - polymère ;
(c) combiner la solution de polymère et d'agent anti-infectieux et le mélange solubilisant-stéroïde pour former un complexe soluble dans l'eau ;
(d) incorporer le complexe dans un système de support pharmaceutiquement acceptable pour former une préparation pharmaceutique stable.

14. Procédé selon la revendication 13, dans lequel le solvant utilisé pour dissoudre ou disperser l'agent anti-infectieux est de l'eau.

15. Procédé selon la revendication 14, dans lequel le polymère augmentant la complexation utilisée est non ionique, et choisi dans le groupe consistant en les alcools polyvinyliques et les polyvinyl pyrrolidones et leurs dérivés.

16. Procédé selon la revendication 15, dans lequel le complexe soluble dans l'eau est lyophilisé.

17. Procédé selon la revendication 16, dans lequel le complexe lyophilisé est incorporé dans un système support convenable.

18. Procédé selon la revendication 15 ou 17, dans lequel le système porteur est de l'eau avec des excipients convenables donnant une préparation pharmaceutique claire pour le traitement oculaire.

19. Procédé selon la revendication 15 ou 17, dans lequel le porteur consiste en de l'eau, un polymère gélifiant et des excipients.

20. Procédé selon la revendication 19, dans lequel le polymère gélifiant est choisi parmi les hydroxy-propyl, méthyl-celluloses, les carbomères et leurs dérivés, la carboxy-méthyl-cellulose ou la méthyl-cellulose ou l'hydroxyéthyl-cellulose.

21. Procédé selon la revendication 15 ou 17, dans lequel le support consiste en une base d'onguent et des excipients.
